## Europäisches Patentamt
## European Patent Office
## Office européen des brevets

(19)

(11) Veröffentlichungsnummer: **0 197 971**
**B1**

(12)

# EUROPÄISCHE PATENTSCHRIFT

(45) Veröffentlichungstag der Patentschrift:
**13.09.89**

(51) Int. Cl.⁴: **A 01 M 13/00**, A 61 L 9/03

(21) Anmeldenummer: **85904813.4**

(22) Anmeldetag: **24.09.85**

(86) Internationale Anmeldenummer:
**PCT/EP 85/00494**

(87) Internationale Veröffentlichungsnummer:
**WO 86/01980 (10.04.86 Gazette 86/08)**

(54) **VERDAMPFERVORRICHTUNG FÜR IN FESTE TRÄGERMATERIALIEN EINGELAGERTE WIRKSTOFFE, WIE PYRETHRUM.**

(30) Priorität: **03.10.84 DE 3436310**

(43) Veröffentlichungstag der Anmeldung:
**22.10.86 Patentblatt 86/43**

(45) Bekanntmachung des Hinweises auf die Patenterteilung:
**13.09.89 Patentblatt 89/37**

(84) Benannte Vertragsstaaten:
**AT BE CH DE FR GB IT LI LU NL SE**

(56) Entgegenhaltungen:
EP-A-0 154 945
WO-A-84/01264
AU-B-441 979
FR-A-2 215 864
FR-A-2 482 420
FR-A-2 530 144

(73) Patentinhaber: **Globol- Werk GmbH, Postfach 1360 Anna- von- Philipp- Strasse 34b, D-8858 Neuburg 1 (DE)**

(72) Erfinder: **VON PHILIPP, Fritz, Auf der Klause 32 1/3, D-8858 Neuburg/Donau (DE)**
Erfinder: **HAUTMANN, Horst, Flachslandenstr. 11, D-8858 Neuburg/Donau (DE)**
Erfinder: **PREGLER, Bernd, Von- Scanzoni- Strasse 1, D-8019 Glonn (DE)**
Erfinder: **SCHIMANSKI, Georg, Bührener Weg 41, D-5800 Hagen 8 (DE)**

(74) Vertreter: **Reinhard, Skuhra, Weise, Leopoldstrasse 51, D-8000 München 40 (DE)**

LIBER, STOCKHOLM 1989

## Beschreibung

Die Erfindung betrifft eine Vorrichtung zum Verdampfen von Wirkstoffen gemäß dem Oberbegriff des Patentanspruchs 1.

Eine Vorrichtung gemäß dem Oberbegriff des Patentanspruchs 1 ist aus der WO-A-84/01264 bekannt. Bei dieser Vorrichtung strömen die Gase vom Brennstofftank durch einen Katalysator in eine Kammer, deren obere Wandung durch eine Wärmeabstrahlplatte gebildet ist. Aus der Kammer strömt die Reaktionswärme durch Öffnungen seitlich aus der Kammer heraus und durch einen Schlitz an der Wirkstoffträgerplatte vorbei. Die durch den Katalysator entwickelte Reaktionswärme bildet bei dieser Vorrichtung eine Strömung, deren Strömungsrichtung mehrmals geändert wird, bevor die Reaktionswärme seitlich der Wirkstoffplatte vorbeiströmt. Die Wärmeabstrahlplatte hat dabei die Funktion, die Reaktionswärme aufzunehmen und an die Wirkstoffträgerplatte direkt weiterzugeben, welche auf der Wärmeabstrahlplatte angeordnet ist. Nachteilig bei dieser Vorrichtung ist der Wechsel der Strömungsrichtung der Reaktionswärme und die Ausbildung von Belüftungsöffnungen an der Oberseite des Gehäuses, was dazu führen kann, daß eine Verwirbelung zwischen durch die Belüftungsöffnung zugeführter Luft und der Reaktionswärme auftritt. Weiterhin besteht das Gehäuse aus zwei Teilen, von denen das untere Teil den Brennstofftank enthält.

Die FR-A-2 482 420 beschreibt eine Vorrichtung zum Verdampfen von Wirkstoffen, bei der brennbare Gase über eine mechanisch zu betätigende Düse austreten und auf einen Katalysator treffen. Über dem Katalysator ist ein Zylinder vorgesehen, der ein Behältnis zum Auflegen von tablettenförmigen Wirkstoffelementen darstellt.

Der Erfindung liegt die Aufgabe zugrunde, eine Vorrichtung der eingangs genannten Art derart auszubilden, daß sie eine bessere Wirkung und eine einfachere Benutzung sicherstellt.

Diese Aufgabe wird erfindungsgemäß durch die Merkmale des Patentanspruchs 1 gelöst.

Weitere Ausgestaltungen der Vorrichtung ergeben sich aus den Unteransprüchen.

Durch die Vorrichtung zum Verdampfen von Wirkstoffen wird dadurch eine bessere Wirkung erzielt, daß der Luftstrom von seitlich im Gehäuse ausgebildeten Belüftungsöffnungen in Form einer stetigen Strömung nach oben strömt und die Wirkstoffplatte direkt beaufschlagt. Die einfache Benutzung der Vorrichtung ergibt sich dadurch, daß in das Fenster des Gehäuses die Wirkstoffplatte einzusetzen ist und darüber hinaus der Brennstofftank auswechselbar von unten in das Gehäuse eingesetzt werden kann. Dadurch, daß das Gehäuse einteilig ausgebildet ist und der Brennertank von unten in das Gehäuse einsetzbar ist, läßt sich die Vorrichtung einfach handhaben und herstellen.

Die Erfindung schafft eine Vorrichtung, bei der durch die direkte Beaufschlagung der Wirkstoffplatte eine zur Aktivierung der Wirkstoffplatte ausreichende Erwärmung sichergestellt ist und trotzdem bei Anwendung brennbarer Stoffe eine Brandgefahr und eine unbeabsichtigte Kontaktnahme mit dem katalytischen Brenner während des Betriebs ausgeschlossen ist.

Die gemäß einer bevorzugten Ausführungsform an der Oberseite des Gehäuses ausgebildete Kammer bewirkt eine Erhöhung des Wirkungsgrades nach Art eines "Kamineffektes".

Ein Ausführungsbeispiel der Erfindung ist in der Zeichnung dargestellt und wird im folgenden näher beschrieben.

Es zeigen:

Fig. 1 eine Verdampfvorrichtung für in feste Trägermaterialien eingebettete Wirkstoffe im Schnitt;

Fig. 2 desgleichen in Richtung der Linie II-II gesehen, wobei Teile der Vorrichtung in Ansicht dargestellt sind ;

Fig. 3 desgleichen von oben gesehen.

Diese Verdampfervorrichtung umfaßt im wesentlichen ein zylindrisches, unten offenes Gehäuse 1 mit einem oberseitig angeordneten Gehäusefenster 2, einem Brennstofftank 3 flacher zylindrischer Form und einem mit dem Brennstofftank 3 zu einer Einheit zusammengefaßten, katalytischen, länglichen Brenner 4. Der Brennstofftank ist samt Brenner 4 in das unten offene Gehäuse 1 eingesteckt und durch eine Rastverbindung gegen unbeabsichtigtes Lösen gesichert.

Hierzu ist am Brennstofftank 3 eine erhabene als umlaufende Sicke ausgebildete Raste 5 angeformt, die in eine innenseitig des Gehäuses 1 angeordnete Nut 6 lösbar eingerastet ist. Dabei bildet die ebene Unterseite des Brennstofftanks 3 mit den unteren Randteilen des Gehäuses 1 die Basis für die Vorrichtung.

Außerdem sind das Gehäuse 1 und der Brennstofftank 3 so ausgebildet, daß der Brennstofftank 3 das unten offene Gehäuse 1 verschließt.

Der Brennstofftank 3 hat eine Füllöffnung 7 zur Aufnahme von flüssigem Brennstoff, insbesondere von Spiritus bzw. Feuerzeugbenzin und ist mit saugfähigem Werkstoff 8, zum Beispiel mit Watte angefüllt.

In der Oberseite des Brennstofftanks 3 ist eine Brennstoff-Verdunstungsöffnung 9 vorgesehen, über welcher der katalytische Brenner gehaltert ist.

Dieser Brenner 4 besteht aus einem länglichen Träger 10 aus porösem feuerfesten und gasdurchlässigen Material, in dem feste Katalysoren, zum Beispiel fein verteiltes Platin, angeordnet ist.

Die Enden des Trägers 10 sind in einem länglichen Halter aus Blech befestigt, der am Brennstofftank 3 unlösbar befestigt ist.

Der längliche, zur Basisseite der Vorrichtung parallel verlaufende Brenner 4 greift in eine an der Oberseite des Gehäuses angeordnete, insbesondere angeformte, zum Brennstofftank 3 hin offene, etwa quaderförmige Kammer 11 ein, die sich zum Gehäuse 1 diametral erstreckt, wobei der Brenner 4 in Richtung seiner Längserstreckung zur Längserstreckung der Kammer 11 gleichgerichtet angeordnet ist.

Damit beim Einstecken des Brennstofftanks 3 samt Brenner 4 in das Gehäuse 1 bzw. beim Überstülpen des Gehäuses 1 über die Brenner-Brennstofftank-Einheit die vorbeschriebene Lage zwangsläufig erreicht wird, können im Gehäuse 1 und am Brennstofftank 3 Orientierungshilfen, insbesondere in Form von achsparallel verlaufenden Geradführungselementen vorgesehen werden.

Um den Brennstofftank 3 samt Brenner 4 zum Zwecke des Füllens mit Brennstoff und/oder zum Zünden des Brenners zum Beispiel mittels eines Streichholzes aus dem Gehäuse beabsichtigter Weise bequem lösen zu können, sind an der Oberseite des Gehäuses 1 zu beiden Seiten der Kammer 11 je eine fingergroße Öffnung 12 vorgesehen, durch die mit den Fingern der Brennstofftank 3 samt Brenner 4 aus dem Gehäuse ausgestoßen werden kann.

In den schmalen Wänden der Kammer 11 sind Belüftungsöffnungen 13 vorgesehen. Über dem Gehäusefenster 2 der Kammer 11 ist in an sich bekannter Weise ein Schutzgitter 14 und ein mindestens an einem Ende offener Einschubkanal zur austauschbaren Aufnahme einer Wirkstoffträgerplatte 15 angeformt.

Ist der aus dem Gehäuse entnommene Brennstofftank 3 mit flüssigem Brennstoff gefüllt und der Brenner 4 entzündet, ist das Gehäuse über die Brenner-Brennstofftank-Einheit zu stülpen und lediglich nur, noch eine Wirkstoffträgerplatte über dem Gehäusefenster zu positionieren.

Zur weiteren Vereinfachung der Handhabe kann in das Gehäuse über dem Brenner eine von außen betätigbare Zündeinrichtung zum Beispiel in Form eines Feuerzeuges mit Reibrad und Feuerstein oder eine piezoelektrische Zündeinrichtung eingefügt werden.

Das Gehäuse 1 samt Kammer 11 und Haltevorrichtung für die Wirkstoffträgerplatten wird vorzugsweise einstückig aus Kunststoff hergestellt, während der Brennstofftank 3 samt Brennerhalterung bevorzugter Weise aus nicht brennbarem Material, insbesondere aus Blech besteht.

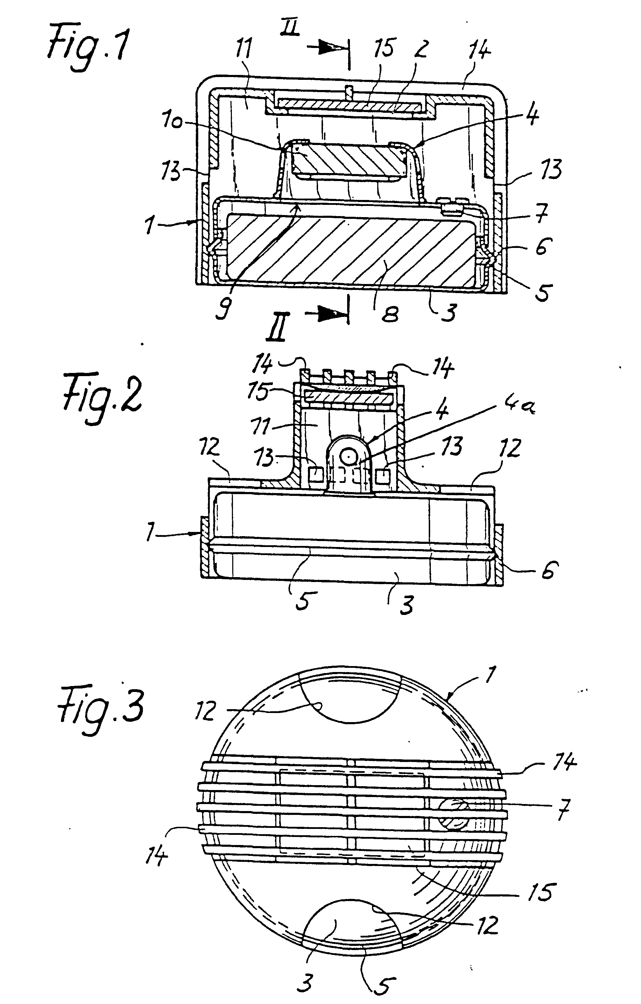

Der bei der erfindungsgemäßen Verdampfervorrichtung verwendete Brenner 4, der auch als Katalysator bezeichnet werden kann, besteht aus einem etwa zylindrischen Träger 10, vorzugsweise in Form eines Drahtnetzes, in welches ein Vlies, vorzugsweise Glasfaservlies, derart eingebracht ist, daß es an der Innenseite des zylindrischen Drahtnetzes zur Anlage kommt. Somit befindet sich das Vlies in einer etwa konzentrischen Anordnung gegenüber dem Drahtnetz-Träger 10 derart, daß das Vlies einen etwa zylindrischen Hohlraum festlegt. Das Vlies selbst ist mit einem katalytischen Element, vorzugsweise Platin, versetzt. Auf diese Weise treten die aus dem Brennstofftank 3 austretenden Gase in das Innere des Brenners 4. Durch das Entzünden der Gase wird erreicht, daß die Gase im Bereich des Brenners einen Glimmvorgang hervorrufen, somit keine Flamme am Brenner 4 selbst entsteht und die durch das Verglimmen der Gase entstandene Hitze direkt nach oben (Fig. 1, 2) zu der Wirkstoffträgerplatte 15 gelangt. Die erhitzten Gase strömen an der Wirkstoffträgerplatte 15 vorbei und bewirken damit eine verstärke Verflüchtigung der Wirkstoffe der Wirkstoffträgerplatte 15. Wesentlich ist, daß die vom Brenner 4 erzeugten Gase direkt auf die Wirkstoffträger 15 wirken; die Wirkstoffplatte 15 ist vorzugsweise in einem Abstand von etwa 8 bis 13 mm zur Oberkante des Brenners 4 angeordnet.

Die erfindungsgemäße Verdampfungsvorrichtung hat somit zum Ziel, die aus dem Brennstofftank 3 austretenden Gase durch den Brenner 4 durchzuleiten, dort erhitzen zu lassen und die erhitzten Gase zu der Wirkstoffträgerplatte 15 zu führen, so daß diese erhitzt wird. Um eine zufriedenstellende Kaminwirkung, d.h. Strömung der erhitzten Gase in Richtung auf die Wirkstoffträgerplatte 15 und an ihr vorbei sicherzustellen, ist die Breite des Gehäusefensters 2 (Fig. 1) größer gewählt als die Breite der Wirkstoffträgerplatte 15, wie deutlich aus Fig. 3 hervorgeht, so daß das erhitzte Gas seitlich an der Wirkstoffträgerplatte 15 an der oberen Seite des Gehäuses 1 austritt. Diese Gase bewirken damit nicht nur eine direkte Erhitzung der Wirkstoffträgerplatte 15, sondern dienen auch als Trägermedium für die von der Platte 15 aufgrund ihrer Erhitzung abgegebenen Wirkstoffpartikel. Die Wirkstoffpartikel werden durch die an der Platte 15 vorbeiströmenden Gase mitgerissen und in die umgebende Atmosphäre verteilt. Die Zufuhr von Sauerstoff zum Brenner 4 wird über die Belüftungsöffnungen 13, die etwa in Höhe der Öffnungen 12 ausgebildet sind, oder über die Öffnungen 12 selbst sichergestellt. Wesentlich ist auch, daß die unterhalb des Brenners 4 ausgebildete schlitzförmige Öffnung an der Oberseite des Brennstofftankes 3 etwa gleiche Länge und Breite hat wie der Brenner 4 selbst oder vorzugsweise eine schmälere Schlitzbreite gegenüber dem Brenner 4. Teil des Brenners 4 ist ein in Fig. 2 mit 4a bezeichnetes Halterungsteil aus Metall, das die schlitzförmige Öffnung des Brennstofftankes 3 umgibt und den Träger 10 in geringem Abstand über der schlitzförmigen Öffnung des Brennertankes 3 hält. Durch diese Halterung 4a ist gewährleistet, daß die aus der Öffnung des Brennertankes 3 austretenden Gase zum Träger 10 gelangen und nicht am Träger 10 vorbei in das Innere des Gehäuses 1 entweichen Vorzugsweise ist die Breite des Halterungselementes 4a (Fig. 2) etwa gleich dem Durchmesser des Trägers 10 gewählt, wodurch eine optimale Zuführung des gasförmigen Brennstoffes vom Brennertank 3 zum Träger 10 gesichert ist.

Um den Abstand zwischen dem Brenner 4 und dem Wirkstoffträger 15 im Funktionszustand der Verdampfungsvorrichtung in der erforderlichen Größenordnung zu halten, ist das Gehäuse 1 zwischen dem unteren, den Brennstofftank 3 aufnehmenden Abschnitt und dem oberen Abschnitt, in welchem der Brenner 4 liegt, abgestuft ausgebildet, wie aus Fig. 2 deutlich zu ersehen ist. Durch die Anlage des Brennstofftankes 3 an der nicht weiter bezeichneten, neben den Öffnungen 12 befindlichen Stufe wird damit der Brenner 4 abstandsmäßig gegenüber dem Wirkstoffträger 15 fixiert.

Ein weiteres wesentliches Merkmal der beschriebenen Verdunstungsvorrichtung besteht darin, daß sie als Folge der verhältnismäßig großen, beispielsweise kreisförmigen Standfläche eine hohe Standfestigkeit hat und gegen ein Umkippen oder versehentliches Umstoßen weitgehend gesichert ist. Wie die Fig. 1 bis 3 zeigen, ist die Standfläche teilweise durch den kreisförmigen unteren Gehäuseabschnitt und die Bodenfläche des Tanks 3 definiert.

Eine kontinuierliche und stetige Strömung heißer Gase zur Wirkstoffträgerplatte 15 und an dieser vorbei ist durch die Sauerstoffzufuhr mittels der unteren Belüftungsöffnungen 13 und die zwischen der Wirkstoffträgerplatte 15 und der Gehäuseoberseite definierten, als Austrittsöffnungen wirkenden Schlitze gegeben. Hierdurch wird eine durch die Sauerstoffzufuhr vom Brenner unbeeinträchtigte, gezielt von unten (Brenner) nach oben (Platte 15) gerichtete Strömung der heißen Gase erhalten, d.h. ein Stau oder eine Verwirbelung und eine mit der Verwirbelung gegebenenfalls verbundene Abkühlung der heißen Gase in dem Raum zwischen dem Brenner 4 und der Platte 15 wird wirksam verhindert.

Schließlich ist bei der erfindungsgemäßen Verdampfungsvorrichtung der Wirkungsgrad bei der Abgabe der Wirkstoffe während ihres Betriebes optimiert. Neben der gerichteten, stetigen Strömung der heißen Gase ist die nach außen bzw. oben liegende Fläche der Wirkstoffplatte 15 mit Ausnahme der vernachlässigbar kleinen Flächen der Stützrippen 14 zur Außenseite freiliegend, wodurch eine durch Gehäuseteile usw. praktisch unbeeinträchtigte Abgabe der Wirkstoffe von der Wirkstoffplatte 15 an die Umgebung erreicht wird.

**Patentansprüche**

1. Vorrichtung zum Verdampfen von Wirkstoffen, die in einer Wirkstoffplatte (15) enthalten sind,
mit einem Gehäuse (1), in welchem ein Brennstofftank (3) und ein katalytischer Brenner (4) angeordnet sind, wobei der katalytische Brenner (4) oberhalb des Brennstofftanks (3) gelagert und durch einen flammenlos wirkenden Brenner (4) gebildet ist,
mit einem über dem katalytischen Brenner (4) im Gehäuse ausgebildeten Fenster (2) zur Aufnahme der Wirkstoffplatte (15), dessen Öffnungsfläche größer als die Fläche der Wirkstoffplatte (15) gewählt ist,
dadurch gekennzeichnet, daß das Fenster (2) unmittelbar über dem Brenner (4) zur Halterung der Wirkstoffplatte direkt über dem Brenner (4) ausgebildet ist, so daß die durch den katalytischen Brenner (4) erhitzten Gase durch das Fenster (2) seitlich an der Wirkstoffplatte (15) vorbei aus dem Gehäuse (1) herausströmen und die vom flammenlos wirkenden Brenner (4) erhitzten Gase die Wirkstoffplatte (15) direkt beaufschlagen und Wirkstoffpartikel der Wirkstoffplatte (15) mitreißen,
daß in der Seitenwand des Gehäuses (1) in der Höhe des katalytischen Brenners (4) Belüftungsöffnungen (13) vorgesehen sind,
daß der Brennstofftank (3) mit einer unter dem katalytischen Brenner (4) liegenden Verdunstungsöffnung (9) versehen ist, daß der Brennstofftank (3) reibschlüssig von unten in das Gehäuse (1) eingesetzt ist, und
daß das Gehäuse (1) aus einem einzigen Teil besteht.

2. Vorrichtung nach Anspruch 1 dadurch gekennzeichnet, daß der Brennstofftank (3) und der katalytische Brenner (4) eine gemeinsame Einheit bilden.

3. Vorrichtung nach einem der vorangehenden Ansprüche, dadurch gekennzeichnet, daß der Brennstofftank (3) die nach unten weisende Öffnung des Gehäuses (1) abschließt.

4. Vorrichtung nach wenigstens einem der vorangehenden Ansprüche, dadurch gekennzeichnet, daß an der Oberseite des Gehäuses eine diametral verlaufende, etwa quaderförmige Kammer (11) ausgebildet ist und daß sich der Brenner (4) zur Längserstreckung der Kammer (11) gleichgerichtet angeordnet ist.

5. Vorrichtung nach wenigstens einem der vorangehenden Ansprüche, dadurch gekennzeichnet, daß über dem Fenster (2) ein Schutzgitter (14) und ein mindestens an einem Ende offener Einschubkanal zur austauschbaren Aufnahme der Wirkstoffplatte (15) angeformt ist.

6. Vorrichtung nach wenigstens einem der vorangehenden Ansprüche, dadurch gekennzeichnet, daß der Brennstofftank (6) eine umlaufend ausgebildete Raste (5) aufweist und daß im Gehäuse (1) eine mit der Raste (5) zusammenwirkende Nut (6) ausgebildet ist.

7. Vorrichtung nach wenigstens einem der vorangehenden Ansprüche, dadurch gekennzeichnet, daß in dem Gehäuse (1) eine Brennerzündeinrichtung vorgesehen ist.

8. Vorrichtung nach wenigstens einem der vorangehenden Ansprüche, dadurch gekennzeichnet, daß der Brennstofftank (3) eine solche Größe hat, daß er die untere Öffnung des Gehäuses (1) verschließt.

9. Vorrichtung nach wenigstens einem der vorangehenden Ansprüche, dadurch gekennzeichnet, daß der Brenner (4) eine längserstreckende Form aufweist und an der Oberseite des Brennstofftanks (3) zur Basisseite des Gehäuses (1) parallel verlaufend vorgesehen ist,
daß das Fenster (2) länglich ausgebildet und zur Längserstreckung des Brenners (4) gleichgerichtet angeordnet ist, und

daß am Brennstofftank (3) und am Gehäuse (1) Führungselemente vorgesehen sind, welche den Brennstofftank (3) gegenüber dem Gehäuse (1) ausrichten.

**Claims**

1. Apparatus for vaporizing active substances which are contained in an active substance plate (15), comprising a housing (1) in which a fuel tank (3) and a catalytic burner (4) are arranged, the catalytic burner (4) being mounted above the fuel tank (3) and formed by a flamelessly acting burner (4),

a window (2) formed in the housing above the catalytic burner (4) for receiving the active substance plate (15), the opening area of said window being made greater than the area of the active substance plate (15),

characterized in that the window (2) is formed directly above the burner (4) for holding the active substance plate directly above the burner (4) so that the gases heated by the catalytic burner (4) flow through the window (2) laterally past the active substance plate (15) out of the housing (1) and the gases heated by the flamelessly acting burner (4) act directly on the active substance plate (15) and entrain active substance particles of the active substance plate (15),

that in the side wall of the housing (1) at the level of the catalytic burner (4) ventilation openings (13) are provided,

that the fuel tank (3) is provided with an evaporation opening (9) lying beneath the catalytic burner (4), that the fuel tank (3) is inserted frictionally locking from below into the housing (1), and

that the housing (1) consists of a single part.

2. Apparatus according to claim 1, characterized in that the fuel tank (3) and the catalytic burner (4) form a common unit.

3. Apparatus according to any one of the preceding claims, characterized in that the fuel tank (3) closes the downwardly directed opening of the housing (1).

4. Apparatus according to at least one of the preceding claims, characterized in that at the upper side of the housing a diametrically extended substantially rightparallelepipedic chamber (11) is formed and that the burner (4) is arranged in the same direction as the longitudinal extent of the chamber (11).

5. Apparatus according to at least one of the preceding claims, characterized in that above the window (2) a protective grating (14) and an insertion passage open at least at one end for exchangeable reception of the active substance plate (15) are formed.

6. Apparatus according to at least one of the preceding claims, characterized in that the fuel tank (3) comprises an encircling detent (5) and that in the housing (1) a groove (6) cooperating with the detent (5) is formed.

7. Apparatus according to at least one of the preceding claims, characterized in that in the housing (1) a burner ignition means is provided.

8. Apparatus according to at least one of the preceding claims, characterized in that the fuel tank (3) has a magnitude such that it closes the lower opening of the housing (1).

9. Apparatus according to at least one of the preceding claims, characterized in that the burner (4) comprises a longitudinally extending form and is provided at the upper side of the fuel tank (3) extending parallel to the base side of the housing (1),

that the window (2) is made elongated and arranged in the same direction as the longitudinal extent of the burner (4), and

that on the fuel tank (3) and the housing (1) guide elements are provided which align the fuel tank (3) with respect to the housing (1).

**Revendications**

1. Dispositif de vaporisation de matières actives contenues dans une plaque (15) de matière active, comportant

un boîtier (1) dans lequel sont disposés un réservoir de combustible (3) et un brûleur catalytique (4), le brûleur catalytique (4) étant placé au-dessus du réservoir de combustible (3) et étant formé d'un brûleur (4) sans flamme,

et une fenêtre (2), formée dans le boîtier au-dessus du brûleur catalytique (4), pour recevoir la plaque (15) de matière active et dont la surface d'ouverture est plus grande que la surface de la plaque (15) de matière active,

caractérisé en ce que la fenêtre (2) est disposée directement au-dessus du brûleur (4) pour maintenir la plaque de matière active directement au-dessus du brûleur (4), de sorte que les gaz qui ont été chauffés par le brûleur catalytique (4) sortent du boîtier (1) par la fenêtre (2) en longeant la plaque (15) de matière active et que les gaz qui ont été chauffés par le brûleur (4) sans flamme viennent directement au contact de la plaque (15) de matière active et entraînent des particules de matière active provenant de la plaque (15) de matière active,

en ce que la paroi latérale du boîtier (1) possède des ouvertures d'aération (13) à la hauteur du brûleur catalytique (4),

en ce que le réservoir de combustible (3) est muni d'une ouverture d'évaporation (9) située sous le brûleur catalytique (4),

en ce que le réservoir de combustible (3) est introduit par le bas dans le boîtier (1), avec frottement,

et en ce que le boîtier (1) est fait d'une seule pièce.

2. Dispositif selon la revendication 1, caractérisé en ce que le réservoir de combustible (3) et le brûleur catalytique (4) forment un seul ensemble.

3. Dispositif selon l'une des revendications précédentes, caractérisé en ce que le réservoir de combustible (3) ferme l'ouverture du boîtier (1) dirigée vers le bas.

4. Dispositif selon l'une au moins des revendications précédentes, caractérisé en ce que, à la face supérieure du boîtier, il est disposé une chambre (11) sensiblement parallélépipédique et s'étendant diamétralement, et en ce que le brûleur (4) est disposé dans la direction longitudinale de la chambre (11).

5. Dispositif selon l'une au moins des revendications précédentes, caractérisé en ce que, au-dessus de la fenêtre (2), il est formé une grille de protection (14) et un conduit d'introduction ouvert à une extrémité au moins et destiné à la mise en place de façon interchangeable de la plaque (15) de matière active.

6. Dispositif selon l'une au moins des revendications précédentes, caractérisé en ce que le réservoir de combustible (3) comprend un cran périphérique (5) et en ce qu'une gorge (6) coopérant avec le cran (5) est formée dans le boîtier (1).

7. Dispositif selon l'une au moins des revendications précédentes, caractérisé en ce qu'un moyen d'allumage du brûleur est prévu dans le boîtier (1).

8. Dispositif selon l'une au moins des revendications précédentes, caractérisé en ce que le réservoir de combustible (3) a des dimensions telles qu'il obture l'ouverture inférieure du boîtier (1).

9. Dispositif selon l'une au moins des revendications précédentes, caractérisé en ce que le brûleur (4) a une forme allongée et s'étend à la face supérieure du réservoir de combustible (3), parallèlement à la base du boîtier (1),

en ce que la fenêtre (2) a une forme allongée et est orientée dans la direction longitudinale du brûleur (4) et

en ce que, sur le réservoir de combustible (3) et sur le boîtier (1), il est prévu des éléments de guidage qui centrent le réservoir de combustible (3) par rapport au boîtier (1).

Fig.1

Fig.2

Fig.3